# EUROPEAN PATENT SPECIFICATION

(11) **EP 1 459 845 B1**
(45) Date of publication and mention of the grant of the patent: **05.12.2007**
(21) Application number: 04250894.5
(22) Date of filing: 19.02.2004
(51) Int. Cl.: B24C 1/06, C23G 1/00, B08B 3/08, A61F 2/30, A61F 2/36

(54) **Surface treatment of metal**
Metalloberflächenbehandlung
Traitement de surface de métaux

(30) Priority: 24.02.2003 GB 0304168
(43) Date of publication of application: 22.09.2004
(73) Proprietor: BENOIST GIRARD SAS, 14201 Hérouville-Saint-Clair Cédex (FR)
(72) Inventor: Jones, Eric, Kildimo, County Limerick (IE)
(74) Representative: Bridge-Butler, Alan James

(56) References cited:
- EP-A- 0 212 929
- WO-A-92/05745
- WO-A-96/16611
- WO-A-96/29030
- GB-A- 2 106 430
- US-A- 5 057 108
- US-A- 5 307 594
- US-A- 5 344 494
- US-A- 5 456 723
- US-A- 5 483 563
- US-A- 6 025 536
- US-A1- 2004 016 651

## Description

This invention relates to the surface treatment of metals to produce a roughened surface and is more particularly, although not exclusively, applicable to the treatment of metal prostheses.

Grits and blasting media of various types are used to roughen the surfaces of prostheses to specific extents of surface roughness to achieve optimum bone interlock features. For a cement-free femoral stem, a peaky surface is preferable to maximise primary stability, whilst sub-surface valleys enable new bone to interlock to provide a more long-term solution for stability. It has been demonstrated (Randelli G. Romano C, Visentin O, : Long term results of Alloclassic-Zweymuller hip prosthesis. JBJS (Br), 79 (suppl II), 238, 1997*)* that surfaces with an average roughness (S_{q}) of approximately 5 -10 µm and peak to valley average (Sₜ) value of approximately 50 - 100 µm meets these requirements.

In order to produce such rough surfaces, blasting conditions and media type need to be more severe than those normally used when a matt or satin finish is required on the prosthesis. Resulting from these extreme conditions is both the embedment of the blasting media in the substrate surface and light attachment to the roughened surface. The latter can be removed relatively easily, but the embedded particles resist the cleaning methods employed and remain attached, and also contribute to the overall surface roughness as proud spikes above the surface. Such contamination has been previously reported (Delaunay C, Bonnomet F, North J, Jobard D, Cazeau C. Kempf J, : Grit-blasted titanium femoral stem in cementless primary total hip arthroplasty. J of Arthroplasty, Vol 16, No. 1, 2001*), (*Bohler M, Kanz F, Schwarz B. Steffan I, Walter A, Plenk H, Knahr K,: Adverse tissue reactions to wear particles from Co-alloy articulations, increased by alumina-blasting particle contamination from cementless Ti-based total hip implants. JBJS Vol 84-B, No. 1, 2002*)*, and identified as a serious concern, with the possibility of both particle detachment with consequential third body wear and inflammatory response in tissues from loose, sharp particles.

US Patent No 5,456,723 describes a metal implant, which has a contact surface roughness of more than 20 microns to provide a good bond between the bone and the implant. This bond is significantly improved by giving the contact surface micro roughness of 2 microns or less. According to the invention this micro roughness is realised by treating a metallic body destined to become the implant with a reducing acid, which attacks the metallic surface to produce the specified micro roughness. This reducing acid may be one of a group of acids including hydrochloric acid, hydrofluoric acid, and a mixture of hydrochloric and sulphuric acids. The reducing acid is preferably made to exert its action on the implant in its boiling state. This acid treatment alone produced the results desired by effectively etching the surface of the metal implant. Sandblasting may be used as a step preceding the reducing acid treatment.

EP 1386 591 A1 describes a method for the manufacture of a prosthetic implant which includes the steps of roughening the surface of the implant by blasting and treating the surface with a solvent which selectively dissolves the blasting particles which are cast steel blasting grains. The surface is treated after blasting by immersion into mechanically stirred acid. The method is designed to define the roughness in the term of R values which is the 2-dimensional contacting method. Such values are, however, not accurate enough to provide the definitions of the peaks and sub-surface values necessary to enable new bone to interlock to provide a long term solution for stability of a prosthesis in the bone. R values are, however, suitable for defining a roughened surface which can, for example, be used with implants which are cemented in in order to give the bone cement a better grip, as referred to in this Application. A disadvantage with this earlier method is that the solvents which selectively dissolve the blasting particles are unlikely to dissolve blasting particles which are sharper and stronger as they are only intended to remove cast steel blasting grains which, in themselves, are not appropriate for providing the peaks and values required for a prosthesis surface which is intended for location in bone without cement.

WO 96/16611 shows a process for improving the surface of devices to be surgically implanted in living bone and refers to a titanium body which has an outside layer. The invention is concerned with removing the outside layer and refers to grit blasting but the material used for blasting is a grit made of titanium or a titanium allow which is intended to avoid contaminating the surface. The invention includes a second acid etching step which is used to produce a surface topography that includes many fine projections having a cone-like aspect. The amount of roughness is, however, not compatible with the requirements of devices for which the present invention is intended.

The present invention is intended to overcome some of the problems set forth above.

According to the present invention a method of forming a roughened, decontaminated surface on a metal cement-free insert portion of a prosthesis by single or multiple blasting of the surface with blasting particles and treating the surface with a solvent to selectively dissolve the blasting particles is characterised in that the blasting particles are chilled cast iron grit of a roughness structure between 180 to 1190 microns applied with a stand off distance of between 10 to 50 mm, blow cleaning the surface by compressed air before treating the surface by ultra-sonic agitation in an acid bath and subsequently rinsing in multiple water baths with ultra-sonic agitation to produce a contamination free surface of substantially S_{q} 5 to 10 microns

Preferably the surface is pickled in 20% nitric acid for 20 to 40 minutes at ambient temperature.

The use of nitric acid (which is an oxidising acid) effectively dissolves the soluble iron grit embedded or loosely held on the implant surface to provide a decontaminated surface.

Other dilute acids may be used that dissolve the iron grit, but do not attack the metallic implant.

For example dilute hydrochloric acid (20%) can be used to remove any insoluble iron salts which may be left after treatment with the nitric acid.

Ultrasonic agitation during the acid treatment process is preferable to loosen partially solubilised iron salts from the surface of the metal.

It has been found that the process is particularly applicable for use with a metal article made from titanium or titanium alloy.

The method may include blasting with G07 chilled iron grit at a pressure of 6.5 bar at 40 cubic metres per hour of air through a 9.5 mm nozzle and 4.8 mm air jet.

The method can also include first blasting with G12 chilled iron grit at 6.5 bar air pressure through a 9 mm nozzle before blasting with the G07 grit.

The blasting time in both cases can be 3 to 4 minutes with a standoff distance between 10 - 50 mm

In any case at least two warm or cold water rinses can be applied after pickling.

The method can be applied to a prosthesis, which has an insertion portion extending from an operative portion, the roughened decontaminated surface being formed on the insertion portion.

A protective cover can be applied to the operative portion and which may be used to carry the prosthesis during blasting.

Grit selection may be determined by the surface hardness of the material and, as mentioned above, for an un-machined (as forged) titanium alloy it is possible to use G 07 grit to obtain the required level of surface roughness.

For machined titanium alloy (the machining operation work hardens the titanium alloy surface) it may be necessary to use two grits, a first blast with a G12 to give a rough, peaky surface. Use of a second blast with G07 grit can be used to cut the peaks down if required.

Stand-off distance may also be reduced in the case of harder materials, and or pressure increased, so that only one grit type needs to be used. The quality of peaky surface may be slightly reduced.

If the hardness is even greater it may be necessary to us an even rougher grade of grit to give the initial cut to the surface, and then refine to the desired surface profile with a less course grit.

Softer surfaces can also be accommodated with a combination of grit selection, pressure, blasting time and stand-off distance.

The acid leach does alter the roughness of the surface by a small amount. This is not, however, by attacking the surface of the metal alloy but by dissolving iron grit embedded in the titanium alloy surface, and which complements to the overall surface roughness.

The method according to the present invention is for the preparation of rough surfaces where the intended implant is affixed into the bone by press-fit (or interference) and is not suitable for the preparation of an implant that is to be affixed by the use of bone cement.

The invention can be performed in various ways and two embodiments will now be described by way of example and with reference to the accompanying drawings in which:
Figure 1 is a pictorial view of a known preferred surface feature with chart showing various parameters;
Figure 2 is a side elevation of a femoral prosthesis to which the method according to the present invention is applied;
Figure 3 is a block diagram showing a first method according to the present invention;
Figure 4 is a block diagram showing another method according to the invention;
Figure 5 is the EDAX profile of the Ti-alloy showing no contamination; and,
Figure 6 is the EDAX profile of Ti-alloy showing contamination by silicon, aluminium and oxygen when a known method of producing a roughened surface is used which does not include the process set forth in the present Application.

Some 20 years clinical history with a cementless, straight-stemmed hip has demonstrated that the typical preferred surface properties are: -
- Sₜ: ten-point height of the surface is in the order of 50 -100 microns
- S_{q}: root mean square deviation of the surface is in the order of 5 - 10 microns.

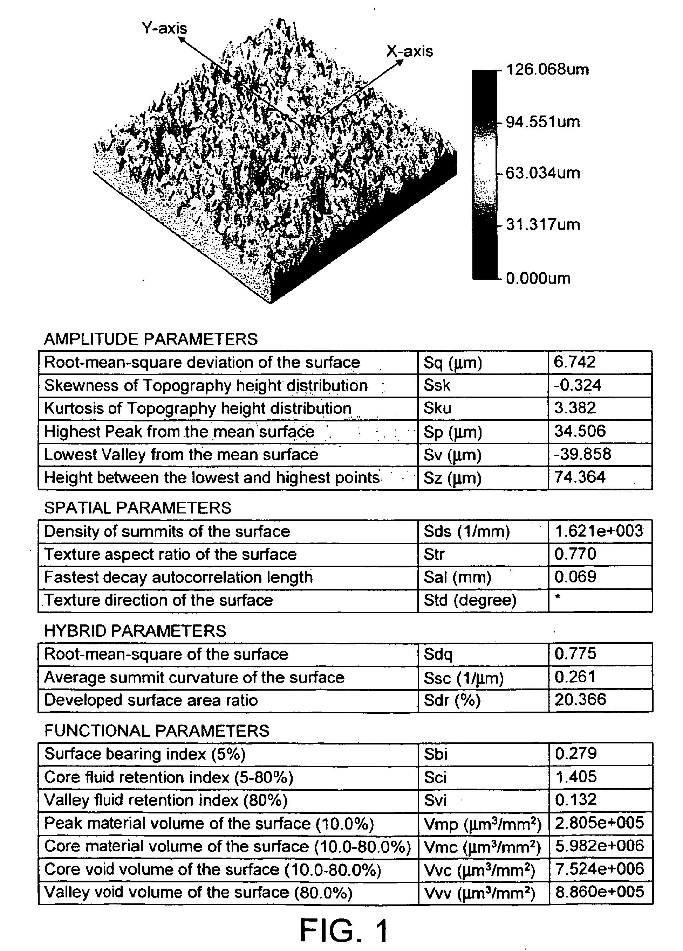

An example of the preferred surface features is shown in Figure 1 and was measured by non-contacting surface profilometry that is a technique that is able to represent a 3-dimensional pictorial display of the surface.

In the preferred surface profile, significant quantities of asperities protrude to give good initial interlock between the stem and the bone and thus imparting early post-operative stability. There is a balance of the valleys to cater for eventual bone on-growth.

As will be seen from Figure 1 the surface topography has proud spikes of bone interlock and rougher valleys for bone on-growth. Round features are avoided making the surface optimal for its intended purpose.

The surface roughness is generally achieved by blasting the surface to be roughened with a blasting medium such as alumina particles.

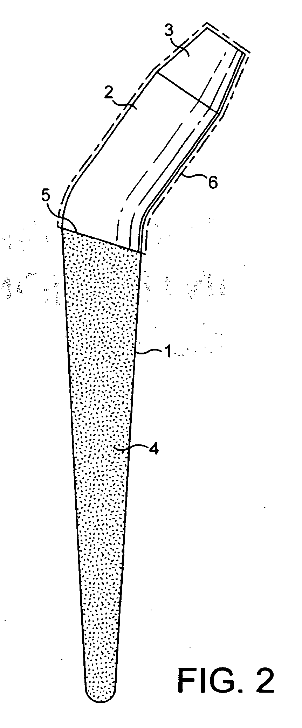

In the method described the invention is applied to a prosthesis which has an insertion portion or stem that is to be inserted into a human bone and which extends from an operative portion. Figure 2 shows a femoral prosthesis to which the present invention can be applied. The prosthesis comprises an insertion portion in the form of a stem 1 which extends from an operative portion provided by a neck 2 on which is a tapered spigot 3 to which a bearing ball can be fitted in known manner. The roughened decontaminated surface indicated by reference numeral 4 is formed on the stem 1.

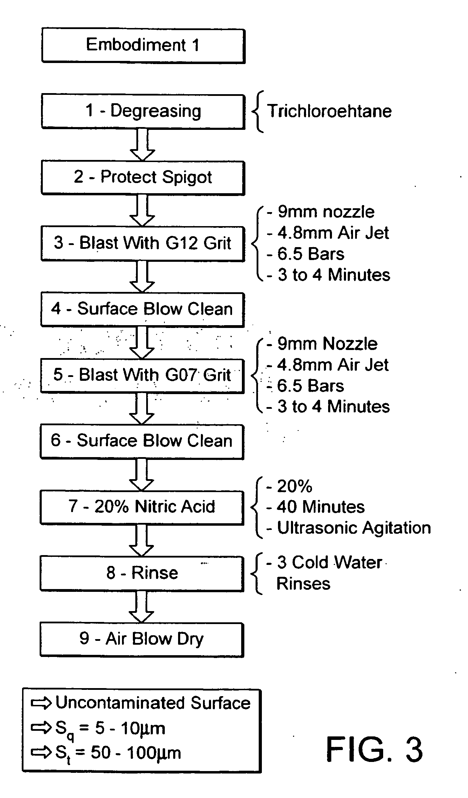

The block diagram shown in Figure 3 illustrates a process according to the invention. Prior to treating the titanium alloy prosthesis the operative portion 2 & 3 of the stem is either dipped or otherwise wrapped to provide a protective covering of polyurethane or other suitable material, which extends down the proximal end of the part of the stem to be treated and which is indicated by reference numeral 5. Thus, all the upper part of the prosthesis above the line 5 is covered by the protective coat, which is indicated by chain lines 6 in Figure 2.

In the first embodiment, prior to the protective coating, however, and as shown in Figure 3, the prosthesis is first degreased, for example, by using trichlorethane an indicated in Box 1. The protective coating is now applied as indicated in Box 2, and the prosthesis is now passed to blasting as indicated in Box 3. In the example being described a Guyson Multiblast six-station machine is used and the prostheses are held in position by clamps, which are attached to the protected portion provided by, for example, the polyurethane covering 6. As the prosthesis is made from machined titanium alloy its outer surface has hardened so, in the method being described the stem 1 is first blasted with G12 chilled iron grit for 3 minutes with a 9 mm nozzle at 6.5 bar air pressure.

The surface is then blow cleaned with air as indicated by Box 4 and is then blasted with G07 chilled iron grit, again for 3 minutes with a 9-mm nozzle at 6.5 bar air pressure as shown in Box 5. The surface is again blown clear by compressed air as shown in Box 6 and the prosthesis, as shown in Box 7 is placed in a bath containing 20% nitric acid for more than 20 minutes and less than 40 minutes with ultrasonic agitation.

The prosthesis is then rinsed in warmed water bath with ultrasonic agitation as indicated in Box 8 and subsequently air blown dry (Box 9)

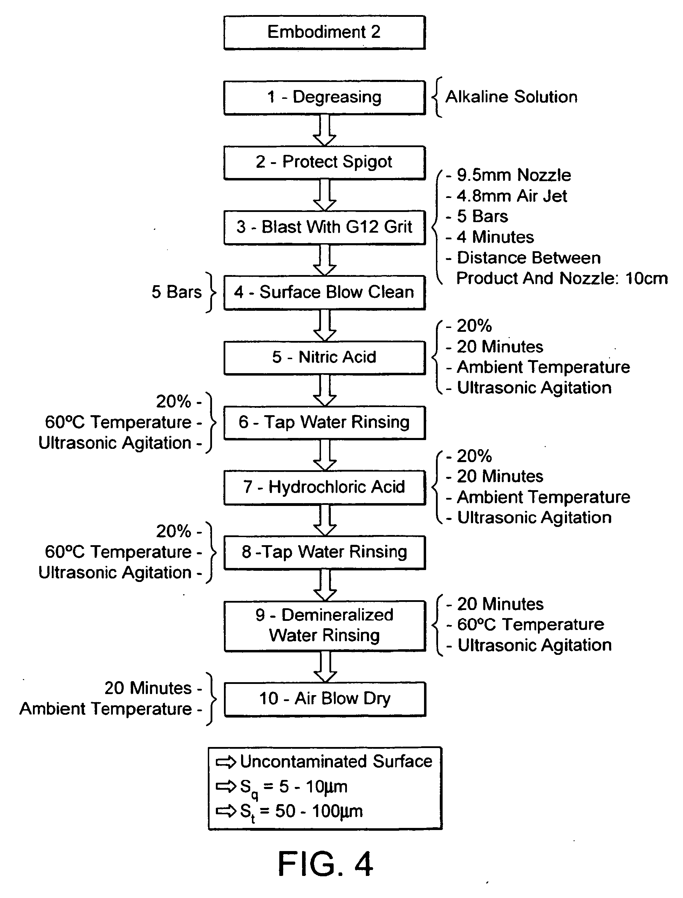

A second embodiment is shown in Figure 4, and, as indicated in Box 1, the product is first degreased using for example an alkaline solution. A protective cover is applied over the spigot, as described with reference to Figure 3, as indicated in Box 2, and the prosthesis is now passed to the blasting station as indicated in Box 3. In the example being described a Guyson Multiblast six-station unit is again used as previously described. In this case a single blast using the G12 chilled iron grit for 4 minutes with applied air pressure of 5 bar and 9.5- mm nozzle is employed.

The surface is then blow cleaned with air at 5 bar as indicated in Box 4, after which it is placed in a bath containing 20% nitric acid for no less than 20 minutes and no more than 40 minutes with continuous ultrasonic agitation as indicated in Box 5. As indicated in Box 6, the component is then rinsed in a bath with warmed tap water for a period of 20 minutes, again with ultrasonic agitation. The process to this point has removed all of the readily solubilised iron salts.

A further step to remove any insoluble salts utilises pickling in a bath of 20% hydrochloric acid for more than 20 minutes and less than 40 minutes with ultrasonic agitation as described in Box 7. The prosthesis is then rinsed in a warmed tap water bath with ultrasonic agitation as indicated in Box 8. A final washing in warm demineralised water again employing ultrasonic agitation completes the cleaning process as indicated in Box 9.

The product is then air dried at ambient temperature for 20 minutes or until dry.

Both embodiments described can be operated in a manual or fully automated mode, or some appropriate combination.

The specification of the chilled iron grit is shown below.

| C | Si | Co | Ni | Cr | Mo | S | P | Pb | Fe |
|---|---|---|---|---|---|---|---|---|---|
| 3.01 | 1.69 | 0.35 | 0.33 | 0.19 | 0.06 | 0.078 | 0.069 | 0.0016 | Bal |

Typical chilled iron grit sizes available for use are:
G07 (180 - 350 microns)
G07 sieved to remove the fraction above 300 microns
G05 (150 - 300 microns)
G12 (250 - 500 microns)
G34 (710 - 1190 microns)

Grit selection is determined by the surface hardness of the material for the desired surface roughness.

For un-machined (as forged) titanium alloy it is possible to use G07 grit only to obtain the required level of surface roughness and the stages shown in Boxes 3 and 4 will not be necessary.

For the machined titanium alloy (the machining operation work hardens the titanium alloy surface) as described above, it may be necessary to use 2 grits.

A first blast with G12 gives a rough, peaky surface.

A second blast with G07 cuts the peaks down.

If the hardness is even greater, it may be necessary to use even rougher grit to give the initial cut into the surface.

The acid leach does alter the roughness of the surface after blasting by a small amount. This is not, however, by attacking the surface of the alloy, but by dissolving the iron grit embedded in the titanium alloy surface. It has been shown that the roughness of the clean surface is unaltered by a further acid wash so indicating that the acid does not chemically attack the metal surface.

The blasting time will depend upon the size of the implant and the metal, but is preferably between 3 to 4 minutes with a stand off distance of 10 - 50 mm.

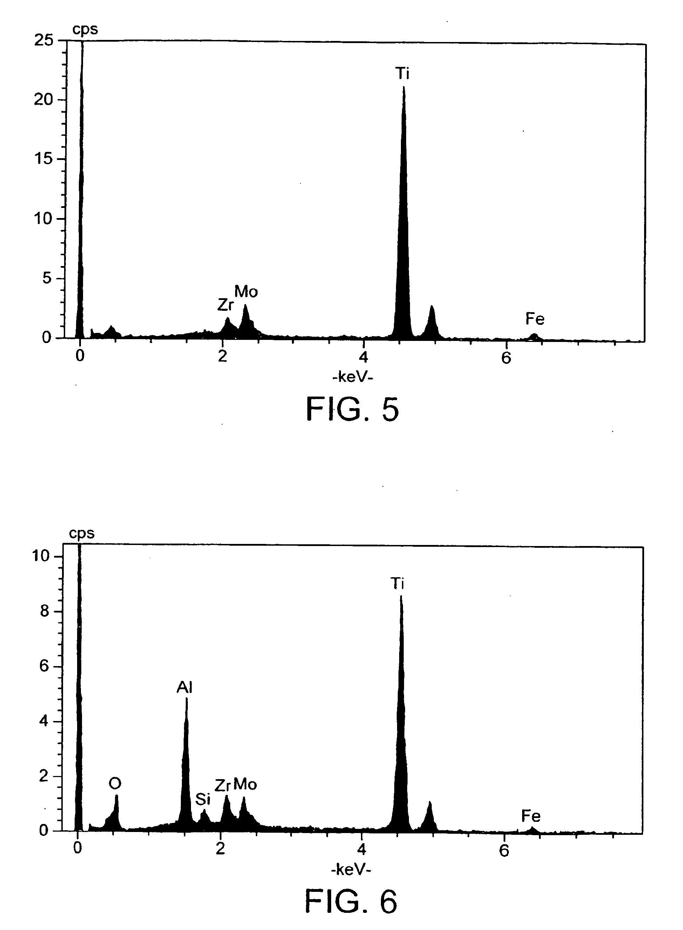

It has been found (using EDAX probing for elemental analysis), as shown in Figure 5, that the surface is substantially uncontaminated, apart from small quantities of zirconium and molybdenum, the nitric acid acting to dissolve the residual iron grit, and this also provides a surface, which, depending upon the resting time can be S_{q} 5 - 10 microns. (As determined using non-contacting surface profilometry.)

Figure 6 is an EDAX profile similar to that shown in Figure 5 of a Ti-alloy but showing additional contamination by oxygen, aluminium and silicon when a known method of producing a roughened surface is used which does not include the process set forth above. Comparison of Figures 5 and 6 clearly indicates the reduction in contamination achieved by the present invention over the previously known process.

## Claims

1. A method of forming a roughened, decontaminated surface on a metal cement-free insert portion of a prosthesis by single or multiple blasting of the surface with blasting particles and treating the surface with a solvent to selectively dissolve the blasting particles **characterised in that** the blasting particles are chilled cast iron grit of a roughness structure between 180 to 1190 microns applied with a stand off distance of between 10 to 50 mm, blow cleaning the surface by compressed air before treating the surface by ultra-sonic agitation in a nitric acid bath and subsequently rinsing in multiple water baths with ultra-sonic agitation to produce a contamination free surface of substantially S_{q} 5 to 10 microns.

2. A method as claimed in claim 1 in which the acid bath pickles the surface in 20% nitric acid for 20 to 40 minutes.

3. A method as claimed in claim 1 or claim 2 which includes blasting with G12 chilled cast iron grit at 6.5 bar air pressure through a 9 mm nozzle.

4. A method as claimed in claim 3 in which the blasting time or times is 3 to 4 minutes with a stand off distance of between 10 to 50 mm.

5. A method as claimed in claims 1 to 4 which includes a second blasting after blow cleaning, the second blasting being with G07 chilled cast iron grit with a 9 mm nozzle at 6.5 bar air pressure for three minutes subsequently again blow cleaning with compressed air before being treated in the acid bath.

6. A method as claimed in any one of the preceding claims which includes treating the surface with dilute hydrochloric acid to remove any insoluble iron salts left after treatment with the nitric acid.

7. A method as claimed in claim 6 which includes blasting with G12 chilled cast iron grit for 4 minutes with applied air pressure of 5 bar and a 9,5 mm nozzle, blow cleaning at 5 bar, placing in the nitric acid bath with ultrasonic agitation, rinsing with warmed tap water with ultrasonic agitation and then pickling in a bath of 20 % Hydrochloric acid and for more than 20 minutes and less than 40 minutes with ultrasound agitation and then rinsing in a warmed tap water with ultrasonic agitation and finally washing in warm demineralised water with ultrasonic agitation.

8. A method as claimed in any one of the preceding claims which includes applying a protective cover to the operative portion.

9. A method as claimed in claim 8 which includes using the protective cover to carry the prosthesis during blasting.

10. A method as claimed in any one of the preceding claims 1 to 9 in which the metal is titanium alloy.

11. A prosthesis having an insertion portion extending from an operative portion and in which a roughened decontaminated surface has been formed by the method set forth in any one of claims 1 to 10.

## Patentansprüche

1. Verfahren zur Bildung einer angerauhten, dekontaminierten Oberfläche auf einem zementfreien Metallinsertionsteil einer Prothese durch einmaliges oder mehrmaliges Strahlen der Oberfläche mit Strahlteilchen und Behandeln der Oberfläche mit einem Lösungsmittel zur selektiven Auflösung der Strahlteilchen, **dadurch gekennzeichnet, daß** die Strahlteilchen Hartgußgrit mit einer Rauhigkeitsstruktur zwischen 180 und 1190 Mikrometern sind, angewendet mit einem Abstand zwischen 10 und 50 mm, die Oberfläche durch Druckluft vor der Behandlung der Oberfläche durch Ultraschallbewegung in einem Salpetersäurebad blasgereinigt und anschließend in mehreren Wasserbädern unter Ultraschallbewegung zur Erzeugung einer kontaminationsfreien Oberfläche mit einer S_{q} von im wesentlichen 5 bis 10 Mikrometern gespült wird.

2. Verfahren nach Anspruch 1, wobei das Säurebad die Oberfläche in 20%iger Salpetersäure für 20 bis 40 Minuten beizt.

3. Verfahren nach Anspruch 1 oder Anspruch 2, das das Strahlen mit G12-Hartgußgrit bei einem Luftdruck von 6,5 bar durch eine 9-mm-Düse umfaßt.

4. Verfahren nach Anspruch 3, wobei die Strahlzeit oder -zeiten 3 bis 4 Minuten mit einem Abstand zwischen 10 und 50 mm beträgt/betragen.

5. Verfahren nach einem der Ansprüche 1 bis 4, das ein zweites Strahlen nach dem Blasreinigen umfaßt, wobei das zweite Strahlen mit G07-Hargußgrit mit einer 9-mm-Düse bei einem Luftdruck von 6,5 bar für drei Minuten stattfindet, wobei anschließend vor der Behandlung im Säurebad erneut mit Druckluft blasgereinigt wird.

6. Verfahren nach einem der vorhergehenden Ansprüche, das die Behandlung der Oberfläche mit verdünnter Salzsäure zur Entfernung jeglicher unlöslicher Eisensalze, die nach der Behandlung mit der Salpetersäure verblieben sind, umfaßt.

7. Verfahren nach Anspruch 6, das das Strahlen mit G12-Hartgußgrit für 4 Minuten mit einem angelegten Luftdruck von 5 bar und einer 9,5-mm-Düse, das Blasreinigen bei 5 bar, das Plazieren in das Salpetersäurebad unter Ultraschallbewegung, das Spülen mit erwärmtem Leitungswasser unter Ultraschallbewegung und dann das Beizen in einem Bad aus 20%iger Salzsäure und für mehr als 20 Minuten und weniger als 40 Minuten unter Ultraschaubewegung und dann das Spülen in erwärmtem Leitungswasser unter Ultraschallbewegung und schließlich das Waschen in warmem demineralisiertem Wasser unter Ultraschallbewegung umfaßt.

8. Verfahren nach einem der vorhergehenden Ansprüche, das das Auftragen eines Schutzüberzuges auf den Operationsteil umfaß.

9. Verfahren nach Anspruch 8, das die Verwendung des Schutzüberzuges zum Halten der Prothese während des Strahlens umfaßt.

10. Verfahren nach einem der vorhergehenden Ansprüche 1 bis 9, wobei das Metall eine Titanlegierung ist.

11. Prothese mit einem Insertionsteil, der von einem Operationsteil ausgeht, und in dem eine angerauhte, dekontaminierte Oberfläche durch ein Verfahren, dargelegt in den Ansprüchen 1 bis 10, gebildet wurde.

## Revendications

1. Procédé pour former une surface rugosifiée décontaminée sur une partie d'insert métallique sans ciment d'une prothèse, par abrasion unique ou multiple de la surface avec des particules abrasives et traitement de la surface avec un solvant pour dissoudre sélectivement les particules abrasives, **caractérisé en ce que** les particules abrasives sont en grenaille de fonte trempée ayant une structure de rugosité comprise entre 180 et 1190 micromètres et sont appliquées avec une distance d'éloignement comprise entre 10 et 50 nm, nettoyage par soufflage de la surface avec de l'air comprimé avant traitement de la surface par agitation ultrasonique dans un bain d'acide nitrique, et ensuite rinçage dans de multiples bains d'eau avec agitation ultrasonique pour produire une surface exempte de contamination ayant une S_{q} sensiblement de 5 à 10 micromètres.

2. Procédé selon la revendication 1, dans lequel le bain d'acide décape la surface dans de l'acide nitrique à 20 % pendant 20 à 40 minutes.

3. Procédé selon la revendication 1 ou la revendication 2, qui comprend une abrasion avec de la grenaille de fonte trempée G12 sous une pression d'air de 6,5 bars passant par une buse de 9 mm.

4. Procédé selon la revendication 3, dans lequel le ou les temps d'abrasion sont de 3 à 4 minutes avec une distance d'éloignement comprise entre 10 et 50 mm.

5. Procédé selon les revendications 1 à 4, qui comprend une deuxième abrasion après nettoyage par soufflage, la deuxième abrasion étant réalisée avec de la grenaille de fonte trempée G07 au moyen d'une buse de 9 mm sous une pression d'air de 6,5 bars pendant 3 minutes, ensuite de nouveau un nettoyage par soufflage avec de l'air comprimé, avant traitement dans le bain d'acide.

6. Procédé selon l'une quelconque des revendications précédentes, qui comprend le traitement de la surface avec de l'acide chlorhydrique dilué pour éliminer tous les sels de fer insolubles restant après traitement avec l'acide nitrique.

7. Procédé selon la revendication 6, qui comprend une abrasion avec de la limaille de fonte trempée G12 pendant 4 minutes sous une pression d'air appliquée de 5 bar et avec une buse de 9,5 mm, un nettoyage par soufflage sous 5 bars, une mise dans le bain d'acide nitrique avec agitation ultrasonique, un rinçage à l'eau du robinet réchauffée avec agitation ultrasonique, puis un décapage dans un bain à 20 % d'acide chlorhydrique pendant plus de 20 minutes et moins de 40 minutes avec agitation ultrasonique et ensuite un rinçage à l'eau du robinet réchauffée avec agitation ultrasonique et finalement un lavage à l'eau déminéralisée chaude avec agitation ultrasonique.

8. Procédé selon l'une quelconque des revendications précédentes, qui comprend l'application d'un revêtement protecteur à la partie opérationnelle.

9. Procédé selon la revendication 8, qui comprend l'utilisation du revêtement protecteur pour transporter la prothèse durant l'abrasion.

10. Procédé selon l'une quelconque des revendications 1 à 9 précédentes, dans lequel le métal est un alliage de titane.

11. Prothèse ayant une partie d'insertion s'étendant depuis une partie opérationnelle et dans laquelle une surface décontaminée rugosifiée a été formée par le procédé présenté dans l'une quelconque des revendications 1 à 10.
